# EUROPÄISCHE PATENTSCHRIFT

(11) **EP 0 491 082 B1**
(45) Veröffentlichungstag und Bekanntmachung des Hinweises auf die Patenterteilung: **12.04.1995**
(21) Anmeldenummer: 90124817.9
(22) Anmeldetag: 19.12.1990
(51) Int. Cl.: A61N 1/05

(54) **Herzschrittmacherleitung mit einem inneren Kanal und mit einem Elektrodenkopf**
Pacemaker lead with an inner duct and with an electrode head
Conduit de stimulateur cardiaque avec un canal intérieur et une tête d'électrode

(43) Veröffentlichungstag der Anmeldung: 24.06.1992
(73) Patentinhaber: Osypka, Peter, Dr. Ing., 79639 Grenzach-Wyhlen (DE)
(72) Erfinder: Osypka, Peter, Dr. Ing., 79639 Grenzach-Wyhlen (DE)
(74) Vertreter: Schmitt, Hans, Dipl.-Ing.

(56) Entgegenhaltungen:
- EP-A- 0 047 013
- EP-A- 0 282 047
- EP-A- 0 368 568

## Beschreibung

Die Erfindung betrifft eine Herzschrittmacherleitung mit einem inneren Kanal mit einem Stilett oder einem Zugelement gemäß dem Oberbegriff des Patentanspruches 1.

Derartige implantierbare Herzschrittmacherleitungen mit im Kerzen verankerbaren Elektrodenköpfen sind in unterschiedlicher Form, beispielsweise mit Hilfe einer an dem Elektrodenkopf befindlichen Schraubwendel oder mit sich verhakenden, borstenartigen Stiften, schirmartigen Konuskörpern usw. bekannt.

Eine Herzschrittmacherleitung gemäß dem Oberbegriff des Patentanspruches 1 ist in der DE-A-2 539 553 beschrieben, wobei zur Verankerung des Elektrodenkopfes eine Schraubwendel dient. Die zur Verankerung des Elektrodenkopfes erforderliche Schraubbewegung kann mit Hilfe des Stilettes durchgeführt werden, welches mit dem schlitzartigen Hohlraum im Bereich des Elektrodenkopfes in Drehrichtung kuppelbar ist, so daß mit dem Stilett die Drehbewegung zum Verankern des Elektrodenkopfes durchgeführt werden kann. Vorteilhaft ist dabei die gute Verankerung und die gute Reizleitung im Herzgewebe. Dies schließt jedoch die Schwierigkeit ein, daß ein Entfernen der implantierten Elektrode nach Monaten oder Jahren, zum Beispiel infolge einer Infektion des Herzschrittmacher-Systemes, äußerst schwierig und in vielen Fällen ohne operative Maßnahmen sogar unmöglich ist.

Diese Schwierigkeit ergibt sich vor allem dadurch, daß man bisher bei solchen Herzschrittmacherleitungen zunächst versucht, am proximalen Ende der Zuleitung, also am Zugang zu der Herzschrittmacherleitung über die Schrittmachertasche, eine Zugkraft aufzubringen, weil an dieser Stelle die Herzschrittmacherleitung am besten zugänglich ist. Da jedoch der häufig wendelförmige Aufbau der eigentlichen Zuleitung in Verbindung mit einer elastischen isolierenden Kunststoffumhüllung Zugkräfte nur in begrenztem Maße zuläßt, muß ein Dauerzug unterhalb dieser Begrenzung an der Herzschrittmacherleitung über ein Rollen- und Gewichtssystem über einen längeren Zeitraum von bis zu mehreren Tagen aufgebracht werden, um zu versuchen, den Elektrodenkopf und dessen Verankerung allmählich durch diese Dauer-Zugkraft aus dem Gewebe zu löschen und zu entfernen. Abgesehen davon, daß dies nicht immer gelingt und dann die schon erwähnte Operation dennoch notwendig wird, kann es auch bei einer Loslösung des Elektrodenkopfes durch diese Dauer-Zugkraft dazu kommen, daß sich der eingewachsene Elektrodenkopf beim Herausziehen von der die eigentliche Zuleitung der Herzschrittmacherleitung bildenden Wendel löst und im Herzen verbleibt. Somit wird auch in diesem Falle eine Herzoperation erforderlich.

Aus EP-A-368 568 ist eine Vorrichtung zum Herausziehen von Herzschrittmacherleitungen bekannt, die ein Zugelement mit einem spreizbaren Teil an dem distalen Ende aufweist. Dabei wird mit diesem spreizbaren Ende an der Innenseite der von einer Wendel gebildeten Herzschrittmacherleitung angegriffen, so daß das vorstehend erläuterte Problem nicht beseitigt wird, sondern die Zugkräfte wiederum auf die Herzschrittmacherleitung selbst ausgeübt werden. Löst sich dabei der eigentliche Elektrodenkopf von dieser die Herzschrittmacherleitung bildenden Wendel, verbleibt er in unerwünschter Weise im Herzen und muß durch eine Herzoperation entfernt werden.

Es besteht deshalb die Aufgabe, eine Herzschrittmacherleitung der gattungsgemäßen Art zu schaffen, bei welcher die Vorteile einer guten Verankerung des Elektrodenkopfe$ im Herzgewebe beibehalten bleiben, die aber bei Bedarf dennoch einfach und leicht entfernt werden kann, ohne daß die Gefahr besteht, daß die Zuleitung zu dem Elektrodenkopf von diesem abgerissen wird, und ohne daß es einer verminderten Dauerzugkraft über mehrere Tage bedarf.

Diese Aufgabe wird bei einer Herzschrittmacherleitung gemäß den Merkmalen des Oberbegriffes des Patentanspruches 1 mit den Mitteln und Maßnahmen des kennzeichnenden Teiles dieses Patentanspruches 1 gelöst.

Muß eine derartige Herzschrittmacherleitung und vor allem ihr Elektrodenkopf aus dem Herzen herausgezogen werden, kann also ein Zugelement oder Stilett vom proximalen Ende her in die Herzschrittmacherleitung und durch deren inneren Kanal eingeführt und am distalen Ende mit dem Hohlraum und dem dort vorgesehenen Vorsprung oder der Hinterschneidung in axialer Richtung formschlüssig verbunden werden, so daß die zum Entfernen notwendigen Zugkräfte unmittelbar an der Verankerung und dem Elektrodenkopf selbst aufgebracht werden können. Somit ist eine Übertragung dieser Zugkräfte über die in axialer Richtung relativ nachgiebige Zuleitung der Herzschrittmacherleitung nicht notwendig und außerdem ist die Gefahr vermieden, daß diese Zuleitung beim Aufbringen solcher Zugkräfte von dem eigentlichen Elektrodenkopf und seiner Verankerung getrennt wird. Dabei genügt es, das Stilett oder Zugelement bis zu dem Hohlraum vorzuschieben und dort formschlüssig mit dem Vorsprung oder der Hinterschneidung zu kuppeln, um dann die gewünschte Zugkraft unmittelbar dort aufbringen zu können, wo sie benötigt wird.

Eine besonders zweckmäßige Art der Kupplung ist in Anspruch 2 angegeben. Es genügt dann nach dem Vorschieben des Stilettes oder Zugelementes eine Drehbewegung, um die formschlüssige Befestigung in axialer Richtung zu erzielen.

Beispielsweise kann als nach innen ragender Vorsprung in dem Hohlraum wenigstens eine Eindellung oder dergleichen exzentrischer Vorsprung vorgesehen sein und das Zugelement kann als Gegenstück einen in der einen Drehrichtung an dem Vorsprung vorbeschiebbaren und durch Drehung den Vorsprung hintergreifenden Kupplungskopf haben. Solche winkel- oder hammerkopfförmigen Kupplungs-Gegenstücke sind einfach herstellbar und wenig störanfällig, gleichzeitig aber sehr effektiv. In gleicher Weise kann natürlich das Stilett oder Zugelement als Gegenstück auch einen abgeflachten, seinerseits eingedellten, Kopf haben, der in der einen Drehrichtung an dem inneren Vorsprung des Hohlraumes vorbeischiebbar ist und durch die Drehbewegung dann mit seiner Einbuchtung, Ausnehmung oder Eindellung den Vorsorung im Inneren des Hohlraumes umgreift.

Eine andere Möglichkeit der Kupplung des Zugelementes mit einem im Inneren des Hohlkörpers befindlichen Kupplungsvorsprung besteht darin, daß das Zugelement zumindest in seinem Kupplungsbereich aus Memory-Metall besteht und nach dem Vorbeiführen an dem vorzugsweise ringförmigen Kupplungsvorsprung durch Erwärmen diesen Vorsprung auf dessen distaler Seite hintergreift. Dabei kann zur Erwärmung des Memory-Metalles entweder schon die Körperwärme oder bevorzugt eine Kupplung des aus Draht oder dergleichen bestehenden Zugelementes mit einer Stromquelle vorgesehen sein.
Eine wiederum abgewandelte Ausführungsform kann darin bestehen, daß als Vorsprünge Gewindegänge in den Hohlraum ragen und das Zugelement oder Stilett an seinem distalen Ende ein dazu passendes Gegengewinde hat. Die zum Kuppeln dienende Drehbewegung kann dann sogar mehrere Drehungen umfassen, um nämlich das Gewinde des Stilettes mit dem Innengewinde in dem Hohlraum zu verbinden.

Der die Kupplung für das Zugelement aufweisende Hohlraum kann als im distalen Endbereich des isolierenden überzuges der Herzschrittmacherleitung angeordnete Hülse ausgebildet sein, an deren distalem Ende der eigentliche Elektrodenkopf, vorzugsweise eine Einschraubwendel, befestigt ist. Einerseits ist eine solche Hülse beim Einführen der Herzschrittmacherleitung zweckmäßig, weil in ihr ein Stilett zum Vorschieben der Herzschrittmacherleitung und ihres Elektrodenkopfes ein gutes Widerlager finden kann und auch die erforderliche Kraft und Bewegung zum Verankern, beispielsweise eine Drehbewegung für eine Einschraubwendel, gut übertragen werden kann und gleichzeitig ist dabei auch eine zugfeste Verbindung zwischen dieser Hülse und dem eigentlichen Ankerteil des Elektrodenkopfes gegeben. Somit kann diese Hülse als Hohlraum eine Doppelfunktion erfüllen, indem sie nämlich einerseits das Implantieren und das Verankern sowie die Reizübertragung erleichtert, aber auch zum Zurückziehen des Elektrodenkopfes nutzbar ist, denn an einer solchen Hülse läßt sich vor allem eine als Kupplungsvorsprung dienende Eindellung bequem anbringen.

Bei einer Herzschrittmacherleitung mit Schraubwendel ist es vorteilhaft, wenn die zur Herstellung der Kupplung zwischen Zugelement und Elektrodenkopf dienende Drehbewegung die entgegengesetzte Drehrichtung aufweist, wie sie für das Einschrauben der Schraubwendel in das Gewebe vorgesehen ist. Wird in dieser Form die Kupplung zwischen dem Zugelement und dem Hohlraum hergestellt, wird gewissermaßen zusätzlich schon eine Drehbewegung an der Schraubwendel aufgebracht, womit diese aus ihrer Verankerungsstellung gelöst werden könnte, falls sie nicht zu stark eingewachsen ist, d.h. die Drehbewegung bei der Kopplung des Zugelementes mit dem Elektrodenkopf beaufschlagt die Schraubwendel in deren Löserichtung.

Eine weitere vorteilhafte Ausnutzung des Hohlraumes mit einer Kupplung für ein Zugelement kann darin bestehen, daß am distalen Ende des Kupplungshohlraumes eine Öffnung zum Abgeben eines Medikamentes an das Herzgewebe vorgsehen ist.

Nachstehend sind Ausführungsbeispiele der Erfindung anhand der Zeichnung näher beschrieben.

Es zeigt in zum Teil schematisierter Darstellung:

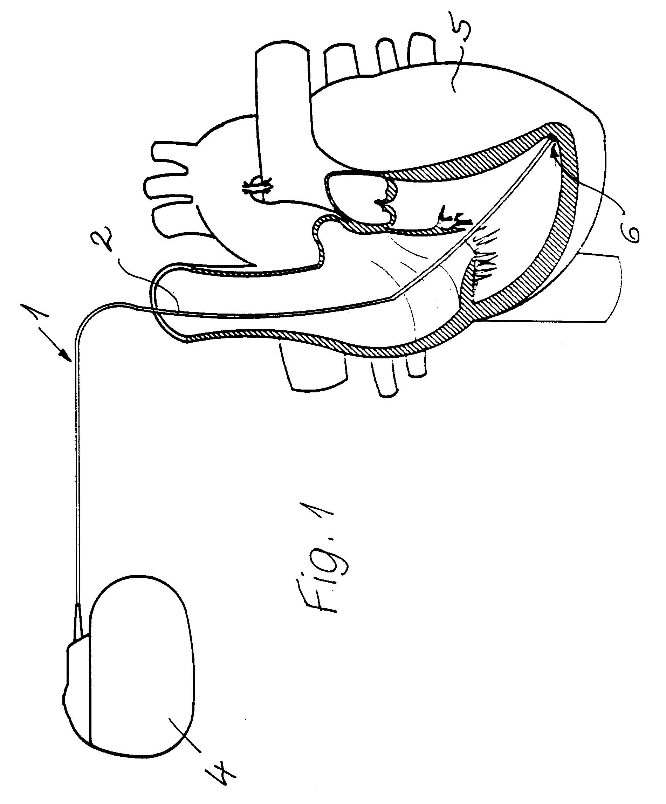

- Fig. 1: eine Herzschrittmacherleitung als Verbindung zwischen dem eigentlichen Herzschrittmacher und der Innenseite eines Herzens, wo ihr Elektrodenkopf im Herzgewebe verankert ist,
- Fig. 2: in vergrößerten Maßstab und im Längsschnitt einen Elektrodenkopf einer Herzschrittmacherleitung gemäß Fig. 1, wobei zum Verankern eine Einschraubwendel vorgesehen ist, die mit Hilfe eines in einen Hohlraum eingreifenden Stilettes in das Herzgewebe eingedreht werden kann, wobei das distale Ende des Stilettes zwischen Einbuchtungen eines Hohlraumes des Elektrodenkopfes greift und so die Drehbewegung übertragbar ist,
- Fig. 3: eine der Fig. 2 entsprechende Darstellung, wobei ein abgewandeltes Stilett mit geändertem Kopf die nach innen gerichteten, als Vorsprünge wirkenden Eindellungen des Hohlraumes hintergreift und zum Herausziehen des Elektrodenkopfes geeignet ist,
- Fig. 4: eine Ansicht der den Hohlraum im Elektrodenkopf bildenden Hülse von deren offener Seite her mit Blick auf die beiden nach innen gerichteten Vorsprünge bildenden Eindellungen,
- Fig. 5: einen Längsschnitt eines abgewandelten Elektrodenkopfes, der einstückig mit einer Hülse verbunden ist, die ein Innengewinde hat, in welches ein Zugelement oder Stilett mit einem passenden Gegengewinde einführbar ist,
- Fig. 6: eine der Fig. 5 entsprechende Darstellung, wobei das Stilett mit seinem Gewinde in das Innengewinde der Hülse des Elektrodenkopfes eingedreht und somit bereit zum Zurückziehen des Elektrodenkopfes ist,
- Fig. 7: ein Stilett aus Memory-Metall, dessen distales Ende U-förmig gekrümmt ist und durch Erwärmung eine Schleife bildet, sowie
- Fig. 8: einen Längsschnitt durch einen Elektrodonkopf mit einem in seiner Hülse befindlichen ringförmigen Vorsprung, in welchen das Stilett gemäß Fig. 7 mit seinem U-förmig gekrümmten Ende einschiebbar ist und welches sich bei der Erwärmung und Verformung hinter dem ringförmigen Vorsprung formschlüssig verhakt.

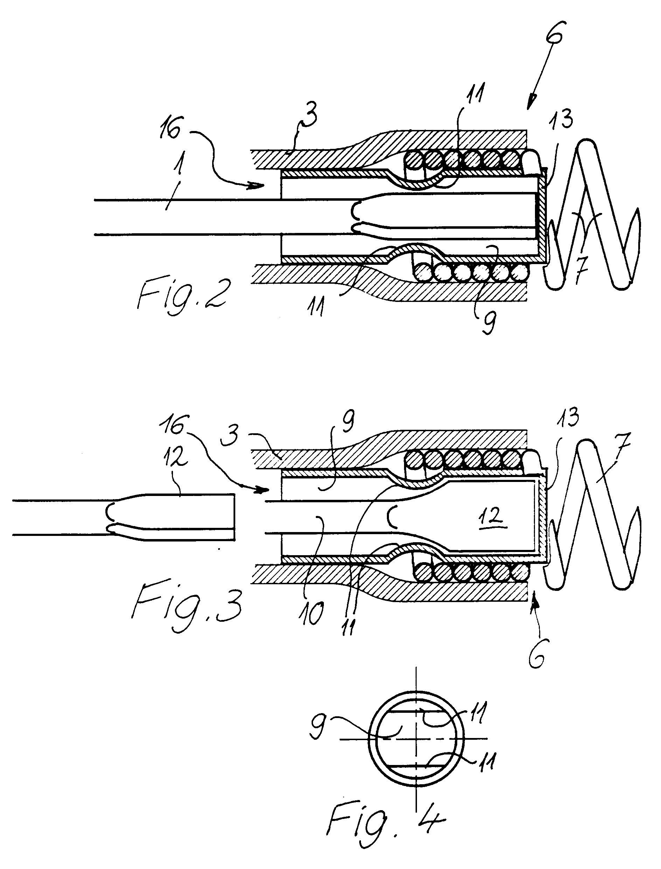

Eine im ganzen mit 1 bezeichnete Herzschrittmacherleitung hat eine Zuleitung 2, die aus einzelnen Windungen wendelförmig aufgebaut sein kann und von einem isolierenden Schlauch 3 überzogen sein kann. Die Zuleitung 2 verbindet dabei einen Herzschrittmacher 4 mit einem im Inneren des Herzens 5 in dessen Gewebe verankerbaren Elektrodenkopf 6, der gemäß den Figuren 2 und 3 oder 5 und 6 oder 8 unterschied-lich gestaltet sein kann, wobei weitere Abwandlungen dieses Elektrodenkopfes 6 möglich sind.

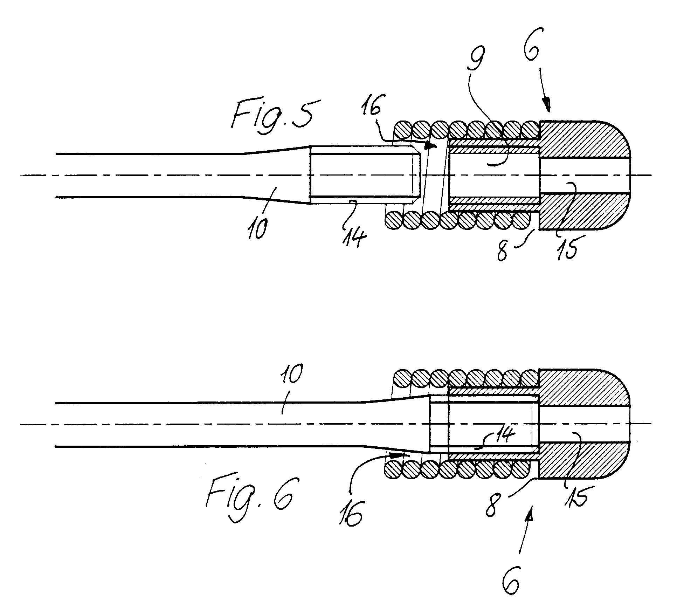

Im Ausführungsbeispiel nach den Figuren 2 und 3 hat der Elektrodenkopf 6 zu seiner Verankerung eine in das Gewebe eindrehbare Schraubwendel 7. Die Elektrodenköpfe 6 gemäß den Figuren 5 und 6 und 8 können sich mit einer Haltekante 8 innerhalb des Herzgewebes verhaken.

Allen Ausführungsbeispielen ist gemeinsam, daß am distalen Ende der Herzschrittmacherleitung 1 ein zu dem Elektrodenkopf 6 gehöriger oder benachbarter Hohlraum 9 vorgesehen ist, der in axialer Richtung vom proximalen Ende der Herzschrittmacherleitung her zugänglich oder offen ist und eine im einzelnen bei den Ausführungsbeispielen jeweils noch zu beschreibende Kupplung für ein vom proximalen Ende der Herzschrittmacherleitung 1 einführbares Zugelement oder Stilett 10 hat. Dabei kann der im Bereich des Elektrodenkopfes 6 angeordnete Hohlraum 9 wenigstens einen, im Ausführungsbeispiel gemäß den Figuren 2 bis 4 zwei einander gegenüberliegende, in sein Inneres ragende Vorsprünge 11 und dadurch Hinterschneidungen haben und das Stilett 10, welches zum Aufbringen einer Zugkraft beim Entfernen des Elektrodenkopfes 6 aus dem Herzen 5 eingesetzt werden soll, weist ein hinter den oder die Vorsprünge 11 in die Hinterschneidung passendes Gegenstück 12 auf, welches durch Drehen in der Lage fixierbar und kuppelbar ist, in der dann Zugkräfte über das Stilett 10 auf den Elektrodenkopf 6 übertragen werden können. In Fig. 2 erkennt man zunächst ein übliches Stilett, welches zwischen die beiden Vorsprünge 11 paßt und zum Eindrehen der Schraubwendel 7 in das Herzgewebe dient. Der flache Kopf des Stilettes ist dabei so lang, daß er von dem distalen Ende des Hohlraumes 9 über die Vorsprünge 11 hinweg reicht.

Gemäß Fig. 3 ist das als Zugelement dienende Stilett 10 mit einem kürzeren Kopf 12 versehen, der nach dem Einschieben bis an das distale Ende oder die Stirnwand 13 des Hohlraumes 9 so verdreht werden kann, daß gemäß Fig. 3 dieser verbreiterte Kopf die Vorsprünge 11 hintergreift und nunmehr eine zugfeste Kupplung bildet. Vor allem Fig. 4 verdeutlicht, daß beim Einschieben des Stilettes 10 mit dem verbreiterten Kopf 12 in der einen Orientierungsrichtung dieser abgeflachte Kopf 12 problemlos zwischen den Vorsprüngen 11 hindurchgeschoben werden kann, wonach er um etwa 90° zu drehen ist, um die in Fig. 3 ersichtliche Gebrauchslage für die Aufbringung einer Rückzugkraft einzunehmen.

Die nach innen ragenden Vorsprünge 11 sind in diesem Ausführungsbeispiel Eindellungen, an denen der Kupplungskopf 12 des Stilettes 10 vorbeischiebbar ist, wonach er in der schon erwähnten Weise durch Drehung gekuppelt werden kann.

Bei dem Ausführungsbeispiel nach den Figuren 5 und 6 ragen als Vorsprünge Gewindegänge in den Hohlraum 9 und das Zugelement oder Stilett 10 hat an seinem distalen Ende ein dazu passendes Gegengewinde 14, welches also durch Drehung mit dem Hohlraum 9 zugfest kuppelbar ist.

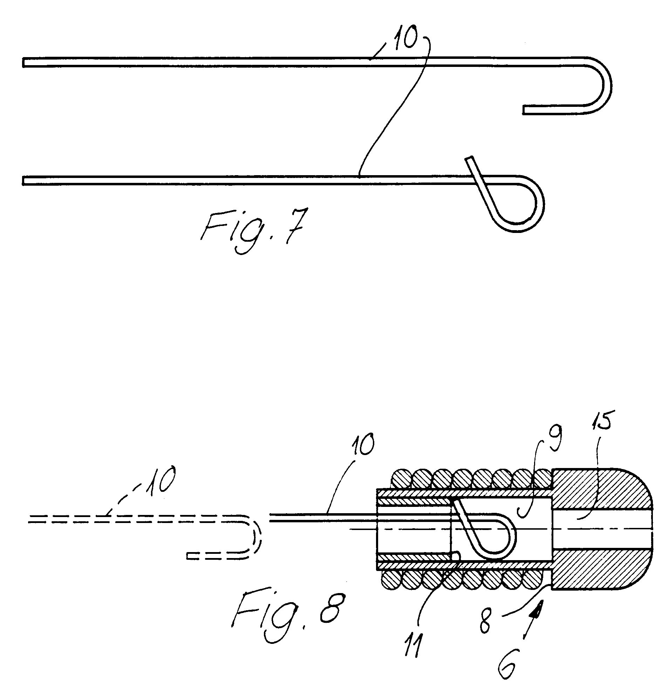

Beim Ausführungsbeispiel nach den Figuren 7 und 8 besteht das Zugelement zumindest in seinem Kupplungsbereich aus Memory-Metall und ist in diesem Kupplungsbereich zunächst U-förmig derart gebogen, daß es durch den ringförmigen Kupplungsvorsprung 11 dieses Ausführungsbeispieles hindurch in einen erweiteren Bereich des Hohlraumes 9 einschiebbar ist. Durch Erwärmung verformt sich dann das Kupplungsende dieses Zugelementes in der dargestellten Weise zu einer Schlaufe, die den ringförmigen Kupplungsvorsprung 11 im Inneren des Hohlraumes 9 formschlüssig hintergreift, so daß auch in diesem Falle wiederum eine hohe Zugkraft unmittelbar auf den Elektrodenkopf 6 ausgeübt werden kann.

Somit ist bei allen Ausführungsbeispielen möglich, eine implantierte Herzschrittmacherleitung 1 aus dem Herzen 5 herauszuziehen und dabei die Zugkraft unmittelbar am Elektrodenkopf 6 aufzubringen.

Es sei noch erwähnt, daß der die Kupplung für das Zugelement aufweisende Hohlraum 9 in all diesen Ausführungsbeispielen als im distalen Endbereich des isolierenden überzuges oder Schlauches 3 der Herzschrittmacherleitung 1 angeordnete Hülse ausgebildet ist, an deren distalem Ende der eigentliche Elektrodenkopf oder die Einschraubwendel 7 befestigt ist. Somit erhält diese Hülse eine Doppelfunktion, weil sie einerseits die Verankerung des Elektrodenkopfes 6 trägt und andererseits den Hohlraum mit dem Kupplungsvorsprung für das Zugelement oder Stilett 10 bildet. In den Ausführungsbeispielen gemäß Fig. 5 bis 8 erkennt man am distalen Ende des Kupplungshohlraumes 9 eine Öffnung 15 zum Abgeben eines Medikamentes an das Herzgewebe, welches Medikament in dem Hohlraum 9 gespeichert werden kann oder durch den inneren Kanal 16 der Zuleitung 2 der Herzschrittmacherleitung 1 zugeführt werden kann. Auch an der stirnseitigen Wandung 13 der Hülse für den Hohlraum 9 gemäß den Figuren 2 und 3 könnte eine solche oder auch eine kleinere Öffnung 15 vorgesehen sein, um ein Medikament allmählich in das Herz abgeben zu können.

Bei allen Ausführungsbeispielen ist es möglich, die implantierte und an dem Elektrodenkopf 6 verankerte Herzschrittmacherleitung 1 aus dem Herzen herauszuziehen und dabei die Zugkraft unmittelbar an dem Elektrodenkopf 6 selbst aufzubringen, so daß die eigentliche Zuleitung 2 diese Zugkräfte nicht übertragen muß und die Verbindung zwischen der Zuleitung 2 und dem Elektrodenkopf 6 durch solche Zugkräfte nicht belastet und gefährtdet wird.
Die Herzschrittmacherleitung 1 mit einer Zuleitung 2 zwischen einem Herzschrittmacher 4 und einem Elektrodenkopf 6 zum Implantieren im Gewebe des Herzens 5 sowie einem inneren Kanal 16 für ein Stilett oder dergleichen hat am distalen Ende einen zu dem Elektrodenkopf 6 gehörigen Hohlraum 9, der in axialer Richtung vom proximalen Ende der Herzschrittmacherleitung 1 her durch den Kanal 16 zugänglich und offen ist und eine Kupplung für ein vom proximalen Ende her einführbares Zugelement oder Stilett 10 hat, so daß die Verankerung des Elektrodenkopfes 6 durch eine unmittelbar an ihm selbst aufgebrachte Zugkraft erforderlichenfalls gelöst werden kann, also keine Reißgefahr im Verlauf der Zuleitung 2 oder an deren Verbindung mit dem Elektrodenkopf 6 besteht. Dabei sind unterschiedliche Möglichkeiten einer Ausgestaltung der Kupplung vorzugsweise mit in das Innere des Hohlraumes 9 ragenden Vorsprüngen 11 möglich, mit denen ein entsprechendes Gegenstuck 12 an dem Zugelement oder Stilett 10 vor allem durch Drehung in Wirkverbindung gebracht werden können.

## Patentansprüche

1. Herzschrittmacherleitung (1) mit einem inneren Kanal (16) mit einem Stilett (10) oder einem Zugelement und mit einem im Inneren des Herzens fixierbaren Elektrodenkopf (6), wobei am distalen Ende der Herzschrittmacherleitung (1) ein zum Elektrodenkopf (6) gehöriger Hohlraum (9) vorgesehen ist, der in axialer Richtung vom proximalen Ende der Herzschrittmacherleitung (1) her zugänglich oder offen ist und eine Kupplung für das vom proximalen Ende der Herzschrittmacherleitung (1) einführbare Stilett (10) oder Zugelement hat, **dadurch gekennzeichnet**, daß der Hohlraum (9) wenigstens einen in sein Inneres ragenden Vorsprung (11) und/oder eine Hinterschneidung hat und daß das Stilett (10) oder Zugelement ein hinter dem Vorsprung (11) oder in die Hinterschneidung passendes Gegenstück (12) aufweist und mit diesem Vorsprung (11) oder der Hinterschneidung kuppelbar ist.

2. Herzschrittmacherleitung nach Anspruch 1, dadurch gekennzeichnet, daß das an dem Stilett (10) oder Zugelement vorgesehene Gegenstück (12) durch Drehen mit dem Vorsprung (11) oder der Hinterschneidung kuppelbar ist.

3. Herzschrittmacherleitung nach Anspruch 1 oder 2, dadurch gekennzeichnet daß als nach innen ragender Vorsprung (11) in dem Hohlraum wenigstens eine Eindellung oder dergleichen exzentrischen Vorsprung vorgesehen ist und daß das Zugelement als Gegenstück (12) einen in der einen Drehrichtung an dem Vorsprung vorbeischiebbaren und durch Drehung den Vorsprung hintergreifenden Kupplungskopf hat.

4. Herzschrittmacherleitung nach Anspruch 1 oder 2, dadurch gekennzeichnet, daß das Zugelement mindestens in seinem Kupplungsbereich aus Memory-Metall besteht und nach dem Vorbeiführen an dem vorzugsweise ringförmigen Vorsprung durch Erwärmung den Vorsprung auf dessen distaler Seite hintergreift.

5. Herzschrittmacherleitung nach Anspruch 4, dadurch gekennzeichnet daß zur Erwärmung des Memory-Metalles eine Verbindung des aus Draht bestehenden Zugelementes mit einer Stromquelle vorgesehen ist.

6. Herzschrittmacherleitung nach Anspruch 1 oder 2, dadurch gekennzeichnet, daß als Vorsprünge Gewindegänge in den Hohlraum (9) ragen und das Zugelement oder Stilett (10) an seinem distalen Ende ein dazu passendes Gegengewinde (14) hat.

7. Herzschrittmacherleitung nach einem der vorstehenden Ansprüchen, dadurch gekennzeichnet, daß der die Kupplung für das Zugelement aufweisende Hohlraum (9) als im distalen Endbereich eines isolierenden Überzuges der Herzschrittmacherleitung (1) angeordnete Hülse ausgebildet ist, an deren distalem Ende der eigentlich Elektrodenkopf, vorzugsweise eine Einschraubwendel (7), befestigt ist.

8. Herzschrittmacherleitung nach einem der Ansprüche 2, 6 oder 7, dadurch gekennzeichnet, daß die zur Herstellung der Kupplung zwischen Zugelement und Elektrodenkopf dienende Drehbewegung die entgegengesetzte Drehrichtung aufweist, wie sie für das Einschrauben einer Schraubwendel in das Gewebe vorgesehen ist.

9. Herzschrittmacherleitung nach einem der Ansprüche 1 bis 8, dadurch gekennzeichnet, daß am distalen Ende des Kupplungshohlraumes (11) eine Öffnung (15) zum Abgeben eines Medikamentes an das Herzgewebe vorgesehen ist.

## Claims

1. A pacemaker lead (1) with an inner duct (16) with a stylet (10) or a pulling element and with an electrode head (6) fixable inside the heart, wherein a cavity (9) belonging to the electrode head (6) is provided at the distal end of the pacemaker lead (1), which cavity is open or is accessible in the axial direction from the proximal end of the pacemaker lead (1) and has a coupling for a stylet 10 or pulling element insertable from the proximal end of the pacemaker lead (1), **characterized in that** the cavity (9) has at least one projection (11) projecting into the cavity interior and/or an undercut and that the stylet (10) or pulling element has a companion member (12) fitting behind the projection (11) or into the undercut and is adapted to be coupled to said projection (11) or undercut.

2. A pacemaker lead as claimed in claim 1, characterized in that the companion member (12) provided on the stylet (10) or pulling member is adapted to be coupled to the projection (11) or undercut by rotation.

3. A pacemaker lead as claimed in claim 1 or claim 2, characterized in that the inward projection (11) in the cavity may be provided in the form of at least one depression or similar eccentric projection and that the pulling element has a companion member (12) in the form of a coupling head which in one orientation can bypass the projection and engages behind the latter by rotation into another orientation.

4. A pacemaker lead as claimed in claim 1 or claim 2, characterized in that the pulling element, at least the coupling zone thereof, consists of memory metal and, after having passed the preferably annular projection, engages behind the distal side of said projection in response to heating.

5. A pacemaker lead as claimed in claim 4, characterized in that for heating the memory metal the pulling element consisting of wire is coupled to a source of electrical energy.

6. A pacemaker lead as claimed in claim 1 or claim 2, characterized in that the projections take the form of threads projecting into the cavity (9) and the pulling element or stylet (10) has at its distal end a mating thread (14).

7. A pacemaker lead as claimed in any one of the preceding claims, characterized in that the cavity (9) having the coupling for the pulling element takes the form of a sleeve arranged in the distal end zone of an insulating cover of the pacemaker lead (1) and attached to the distal end of said sleeve is the actual electrode head, preferably a helix (7).

8. A pacemaker lead as claimed in any one of claims 2, 6 or 7, characterized in that the rotary motion serving to establish the coupling between pulling element and electrode head has a direction of turn counter to that for screwing a helix into the tissue.

9. A pacemaker lead as claimed in any one of claims 1 to 8, characterized in that an opening (15) for discharging a medicament to the tissue of the heart is provided at the distal end of the coupling cavity (11).

## Revendications

1. Conduit de stimulateur cardiaque (1) comprenant un canal intérieur (16), un stylet (10) ou un élément de traction et une tête d'électrode (6) qui peut être fixée à l'intérieur du coeur, cependant qu'il est prévu, à l'extrémité distale du conduit de stimulateur cardiaque (1), une cavité (9) qui fait partie de la tête d'électrode (6), qui est accessible dans la direction axiale depuis l'extrémité proximale du conduit de stimulateur cardiaque (1) ou qui est ouverte dans cette direction, et qui comporte un accouplement destiné au stylet (10) ou à l'élément de traction, lequel peut être introduit depuis l'extrémité proximale du conduit de stimulateur cardiaque (1), caractérisé par le fait que la cavité (9) comporte au moins une partie (11) qui fait saillie dans son volume intérieur et/ou une contre-dépouille, et par le fait que le stylet (10) ou l'élément de traction présente une pièce conjuguée (12) qui s'ajuste derrière la partie en saillie (11) ou dans la contre-dépouille, et qu'il peut être accouplé à cette partie en saillie (11) ou à la contre-dépouille.

2. Conduit de stimulateur cardiaque selon la revendication 1, caractérisé par le fait que la pièce conjuguée (12) qui est prévue sur le stylet (10) ou sur l'élément de traction peut être accouplée par rotation à la partie en saillie (11) ou à la contre-dépouille.

3. Conduit de stimulateur cardiaque selon la revendication 1 ou 2, caractérisé par le fait qu'il est prévu dans la cavité, pour servir de partie (11) en saillie vers l'intérieur, au moins un bossage ou une partie en saillie excentrique similaire, et par le fait que l'élément de traction comporte, pour servir de pièce conjuguée (12), une tête d'accouplement qui peut passer devant la partie en saillie lorsqu'elle a tourné dans l'un des sens de rotation et qui vient en prise par derrière avec la partie en saillie grâce à une rotation.

4. Conduit de stimulateur cardiaque selon la revendication 1 ou 2, caractérisé par le fait que l'élément de traction est constitué par un métal à mémoire, du moins dans sa zone d'accouplement, et qu'après être passé devant la partie en saillie, laquelle est de préférence annulaire, et grâce à un chauffage, il vient en prise par derrière avec la partie en saillie sur le côté distal de celle-ci.

5. Conduit de stimulateur cardiaque selon la revendication 4, caractérisé par le fait qu'en vue du chauffage du métal à mémoire, il est prévu une liaison avec une source de courant pour l'élément de traction qui est constitué par un fil.

6. Conduit de stimulateur cardiaque selon la revendication 1 ou 2, caractérisé par le fait que des spires de filetage qui servent de partie en saillie pénètrent dans la cavité (9), et que l'élément de traction ou le stylet (10) comporte à son extrémité distale un filetage conjugué (14) qui s'ajuste à ses spires.

7. Conduit de stimulateur cardiaque selon l'une des revendications précédentes, caractérisé par le fait que la cavité (9) qui comporte l'accouplement destiné à l'élément de traction est réalisée sous la forme d'un manchon qui est disposé dans la région de l'extrémité distale d'un revêtement isolant du conduit de stimulateur cardiaque (1) et à l'extrémité distale duquel est fixée la tête d'électrode proprement dite (6) qui est de préférence une spire à visser (7).

8. Conduit de stimulateur cardiaque selon l'une des revendications 2, 6 et 7, caractérisé par le fait que le déplacement en rotation qui sert à réaliser l'accouplement entre l'élément de traction et la tête d'électrode présente un sens de rotation opposé à celui qui est prévu pour le vissage dans les tissus d'une spire à visser.

9. Conduit de stimulateur cardiaque selon l'une des revendications 1 à 8, caractérisé par le fait qu'à l'extrémité distale de la cavité d'accouplement (9) est prévue une ouverture (15) qui est destinée à amener un médicament aux tissus cardiaques.
